# EUROPEAN PATENT SPECIFICATION

(11) **EP 4 147 566 B1**
(45) Date of publication and mention of the grant of the patent: **02.07.2025**
(21) Application number: 22171160.9
(22) Date of filing: 02.05.2022
(51) Int. Cl.: A01G 22/15, A01G 24/10, A01G 24/12, A01G 31/00, A01G 31/02, A01G 22/00, A61K 36/23, C05G 1/00, C05G 3/00

(54) **CULTURE MEDIUM COMPOSITION FOR INCREASING AMOUNTS OF MADECASSOSIDE AND ASIATICOSIDE IN CENTELLA ASIATICA AND METHOD OF PREPARING SAME**
KULTURMEDIUMZUSAMMENSETZUNG ZUR ERHÖHUNG DER MENGE VON MADECASSOSID UND ASIATICOSID IN CENTELLA ASIATICA UND VERFAHREN ZU IHRER HERSTELLUNG
COMPOSITION DE MILIEU DE CULTURE POUR AUGMENTER LES QUANTITÉS DE MADÉCASSOSIDE ET D'ASIATICOSIDE DANS CENTELLA ASIATICA ET SON PROCÉDÉ DE PRÉPARATION

(30) Priority: 14.09.2021 KR 20210122402
(43) Date of publication of application: 15.03.2023
(73) Proprietor: Korea Institute of Science and Technology, Seoul 02792 (KR)
(72) Inventor: KIM, Ho Youn, 25451 Jeollabuk-do (KR); NHO, Chu Won, 25451 Jeollabuk-do (KR); PARK, Jai Eok, 25451 Jeollabuk-do (KR)
(74) Representative: advotec.

(56) References cited:
- WO-A1-2005/112607
- GB-A- 1 318 698
- KR-A- 20200 104 158
- RU-C1- 2 070 785
- US-A1- 2020 154 652

## Description

### BACKGROUND

### (a) Technical Field

The present disclosure relates to a culture medium composition for increasing the amounts of madecassoside and asiaticoside in *Centella asiatica* and a method of preparing the same.

### (b) Background Art

*Centella asiatica* L. Urban is a creeping perennial herb belonging to the family *Umbelliferae,* and is native to Madagascar, Africa because it mainly grows in hot and humid places, but also grows widely in the coastal areas of the Indian Ocean or in hot and humid places in Southwest Asia and Central and South America (Hausen, Contact Dermatitis. 29:175-179, 1993). In Korea, it grows in groups in wetlands on Jeju Island and the southern islands.

*Centella asiatica* has been used medicinally for a long time in traditional therapies in India and other parts of Asia (Brinkhaus et al., Phytomedicine. 7:427-448, 2000). Asiaticoside and madecassoside, which are the main ingredients thereof, are pentacyclic triterpene glycosides belonging to the α-amyrin-ursolic acid group, and show activity for the treatment of skin wounds and chronic ulcers (Bonte et al., Plant Medica. 60:133-135, 1993). Additionally, these materials have been used to treat leprosy because they dissolve the waxy capsule of *Mycobacterium leprae* (Vogel et al., Acta Therapeutica. 16:285-296, 1990). In particular, it is known that asiaticoside has antibacterial activity and is thus effective at treating not only skin wounds and gastric ulcers, but also various skin diseases, mental disorders, tuberculosis, venous disease, dementia, and the like (Kim et al., Korean Journal of Medicinal Crop Science. 10:375-378, 2002). It is recently known that water-soluble extracts of *Centella asiatica* contain asiaticoside, madecassic acid, and asiatic acid, which are the main ingredients in herbs, in respective amounts of 40%, 30%, and 30%, and also that these materials have the ability to regenerate skin tissue to thus promote collagen production. In clinical practice, such materials are utilized as major ingredients for ointments for wound healing (Kim et al., Korean Journal of Medicinal Crop Science. 10:375-378, 2002). In this way, *Centella asiatica* is a medicinal plant that is variously used as a skin therapeutic agent, a wound-healing agent, a memory enhancer, a tonic agent, and the like, so demand therefor for use as a material for medicaments and functional cosmetics is also increasing in Korea, but the main habitat thereof is limited to tropical and subtropical regions, and there are difficulties in cultivation and harvesting in Korea.

Since *Centella asiatica* mainly grows in hot and humid places, it seldom grow spontaneously in Korea, except for Jeju Island and the southern islands, which belong to the warm temperate zone, and thus the collection of naturally growing *Centella asiatica* and use of the same as a material for a therapeutic agent are limited. Furthermore, due to environmental pollution caused by rapid industrialization and damage to habitat due to land development, the natural habitat, populations, and numbers of plants have decreased greatly, making it more difficult to collect them, and thus these plants are almost entirely dependent on imports.

Moreover, *Centella asiatica* produced in Korea mainly grows in open fields, which makes it difficult to supply *Centella asiatica* having metabolites above a certain level because the production amounts and functional ingredients vary greatly depending on the season and harvest time.

Since the amounts of the above-mentioned useful materials synthesized in the *Centella asiatica* plant are low, artificial synthesis has been investigated, but the structure is complicated and synthesis is expensive and difficult, so various studies to increase the amounts of materials synthesized in the plant are ongoing.

### [Citation List]

### [Patent Literature]

(Patent Document 1) Korean Patent Application Publication No. 10-2017-0066878
(Patent Document 2) KR 2020 0104158 A discloses a culture medium composition for hydroponic cultivation of *Centella asiatica.*

### SUMMARY OF THE DISCLOSURE

Accordingly, the present inventors have studied novel *Centella asiatica* cultivation technology capable of increasing the amounts of madecassoside and asiaticoside contained in *Centella asiatica* using hydroponic cultivation, thus leading to the present invention.

Hydroponic cultivation is a system that supplies nutrients and water necessary for plant growth, and enables control of the root zone. Compared to a conventional cultivation method, when hydroponically cultivated, *Centella asiatica* may be produced in constant amounts, and fortification of various specific ingredients may be induced. Particularly, the researchers of the present invention have developed a culture medium composition and a cultivation method capable of speeding the growth of *Centella asiatica* and increasing the amounts of particular ingredients therein.

Accordingly, an aspect of the present invention is to provide a culture medium composition for hydroponic cultivation capable of speeding the growth of *Centella asiatica* and increasing the amounts of particular ingredients therein.

Another aspect of the present invention is to provide a method for cultivation of *Centella asiatica* using the culture medium composition.

Still another aspect of the present invention is to provide *Centella asiatica* grown using the method for cultivation of *Centella asiatica.*

Yet another aspect of the present invention is to provide a *Centella asiatica* extract containing madecassoside and asiaticoside in large amounts from the cultivated *Centella asiatica.*

The objects of the present invention are not limited to the foregoing. The objects of the present invention will be able to be clearly understood through the following description and to be realized by the means described in the claims and combinations thereof.

In order to accomplish the above objects, the present invention provides the following composition and a method for preparing the same.

An aspect of the present invention provides a culture medium composition for hydroponic cultivation of *Centella asiatica,* the composition including a macroelement group including phosphorus (P), nitrogen (N), potassium (K), calcium (Ca), magnesium (Mg), and sulfur (S), the weight ratio of phosphorus (P), nitrogen (N), potassium (K), calcium (Ca), magnesium (Mg), and sulfur (S) being 1:3.0-6.0:4.8-7.0:3.0-5.2:0.4-1.7:1.0-3.0.

The culture medium composition according to an aspect of the present invention may include a microelement group including iron, boron, manganese, zinc, copper, and molybdenum.

In the culture medium composition according to an aspect of the present invention, the microelement group may include 3.00 to 7.00 ppm of iron, 0.30 to 1.00 ppm of boron, 0.30 to 1.00 ppm of manganese, 0.10 to 0.30 ppm of zinc, and 0.01 to 0.05 ppm of molybdenum, based on the total weight of the composition.

In the culture medium composition according to an aspect of the present invention, the macroelement group may include, based on 100 parts by weight thereof, 19 to 31 parts by weight of nitrogen, 3 to 10 parts by weight of phosphorus, 30 to 40 parts by weight of potassium, 20 to 30 parts by weight of calcium, 3 to 10 parts by weight of magnesium, and 8 to 16 parts by weight of sulfur.

Another aspect of the present invention provides a method for hydroponic cultivation of *Centella asiatica,* the method including supplying the culture medium composition described above to the roots of *Centella asiatica.*

In the method for hydroponic cultivation according to another aspect of the present invention, the hydroponic cultivation may be performed using a deep flow technique (DFT).

In the method for hydroponic cultivation according to another aspect of the present invention, the method may be performed under light of 5000 to 8000k radiated in an amount of 100 to 300 µmol/m²s.

In the method for hydroponic cultivation according to another aspect of the present invention, the method may be performed under daylength conditions of 10 to 16 hours.

In the method for hydroponic cultivation according to another aspect of the present invention, carbon dioxide may be supplied at a concentration of 700 to 900 ppm only during an illumination period.

In the method for hydroponic cultivation according to another aspect of the present invention, the culture medium composition may be circulated from a culture medium tank to a culture medium recovery tank, and a nutrient solution may be supplied to the roots of *Centella asiatica* during circulation.

In the method for hydroponic cultivation according to another aspect of the present invention, the concentration of the culture medium composition may be EC 0.3 to 2.8.

In the method for hydroponic cultivation according to another aspect of the present invention, the concentration of the culture medium may be increased by EC 0.3 to 0.7 at weekly intervals from a concentration of EC 0.3 to 0.7 after transplanting *Centella asiatica.*

Still yet another aspect of the present invention provides madecassoside isolated from *Centella asiatica* produced using the method for hydroponic cultivation described above.

A further aspect of the present invention provides asiaticoside isolated from *Centella asiatica* produced using the method for hydroponic cultivation described above.

### BRIEF DESCRIPTION OF THE DRAWINGS

The above and other features of the present invention will now be described in detail with reference to certain exemplary embodiments thereof illustrated in the accompanying drawings, which are given hereinbelow by way of illustration only, and thus are not limitative of the present invention, and wherein:
FIG. 1A shows the fresh weight of *Centella asiatica* during 6-week cultivation in Example 1 of the present invention;
FIG. 1B shows the number of leaves of *Centella asiatica* during 6-week cultivation in Example 1 of the present invention;
FIG. 2 shows the results of the amounts of functional ingredients when using the culture medium of Example 2 of the present invention; and
FIG. 3 shows the previous analysis results for the functional ingredients of *Centella asiatica* collected in Korea.

### DETAILED DESCRIPTION

Unless otherwise specified, all numbers, values, and/or representations that express the amounts of ingredients, reaction conditions, and compositions used herein are to be taken as approximations including various uncertainties affecting measurement that inherently occur in obtaining these values, among others, and thus should be understood to be modified by the term "about" in all cases. Furthermore, when a numerical range is disclosed in this specification, the range is continuous, and includes all values from the minimum value of said range to the maximum value thereof, unless otherwise indicated. Moreover, when such a range pertains to integer values, all integers including the minimum value to the maximum value are included, unless otherwise indicated.

In the present specification, when a range is described for a variable, it will be understood that the variable includes all values including the end points described within the stated range. For example, the range of "5 to 10" will be understood to include any subranges, such as 6 to 10, 7 to 10, 6 to 9, 7 to 9, and the like, as well as individual values of 5, 6, 7, 8, 9 and 10, and will also be understood to include any value between valid integers within the stated range, such as 5.5, 6.5, 7.5, 5.5 to 8.5, 6.5 to 9, and the like. Also, for example, the range of "10% to 30%" will be understood to include subranges, such as 10% to 15%, 12% to 18%, 20% to 30%, etc., as well as all integers including values of 10%, 11%, 12%, 13% and the like up to 30%, and will also be understood to include any value between valid integers within the stated range, such as 10.5%, 15.5%, 25.5%, and the like.

Hereinafter, a detailed description will be given of the present invention.

*Centella asiatica* is mainly mass-cultivated in open fields, but the production amount and functional ingredients thereof vary greatly depending on the season and harvest time. Plants vary not only in growth but also in the amounts of secondary metabolites depending on the cultivation environment and system. However, it was difficult to find examples of research on hydroponic cultivation systems in smart farms for the purpose of commercial cultivation of *Centella asiatica.* Hydroponic cultivation pertains to systems that supply nutrients and water necessary for plant growth and enable control of the root zone. Compared to a conventional cultivation method, when hydroponically cultivated, *Centella asiatica* may be produced in constant amounts, and fortification of various specific ingredients may be induced. Accordingly, in the present invention, a culture medium composition and a cultivation method capable of speeding the growth of *Centella asiatica* and increasing the amounts of particular ingredients therein were developed. Based on the results of application of various composition ratios of essential minerals necessary for the growth of *Centella asiatica,* the three results exhibiting the best growth were selected, cultivation was conducted for 6 weeks at the three composition ratios, and a culture medium composition that was very effective for increasing the amounts of functional ingredients was determined. As a result, it was confirmed that the number of leaves was significantly increased in the NS2 culture medium in terms of growth, and also that the amounts of functional ingredients were significantly increased in the above culture medium.

Hereinafter, various aspects of the present invention will be described.

An aspect of the present invention pertains to a culture medium composition for hydroponic cultivation of *Centella asiatica,* the composition including a macroelement group composed of phosphorus (P), nitrogen (N), potassium (K), calcium (Ca), magnesium (Mg), and sulfur (S), the weight ratio of phosphorus (P), nitrogen (N), potassium (K), calcium (Ca), magnesium (Mg), and sulfur (S) being 1:3.0-6.0:4.8-7.0:3.0-5.2:0.4-1.7:1.0-3.0. When the above conditions are satisfied, the amounts of the active madecassoside and asiaticoside ingredients are high.

In the culture medium composition according to an aspect of the present invention, the culture medium composition for hydroponic cultivation of *Centella asiatica* includes a microelement group composed of iron, boron, manganese, zinc, copper, and molybdenum.

In the culture medium composition according to an aspect of the present invention, the microelement group includes 3.00 to 7.00 ppm of iron, 0.30 to 1.00 ppm of boron, 0.30 to 1.00 ppm of manganese, 0.10 to 0.30 ppm of zinc, and 0.01 to 0.05 ppm of molybdenum, based on the total weight of the composition. When the above conditions are satisfied, the amounts of the active madecassoside and asiaticoside ingredients are high.

In the culture medium composition according to an aspect of the present invention, the macroelement group includes, based on 100 parts by weight thereof, 19 to 31 parts by weight of nitrogen, 3 to 10 parts by weight of phosphorus, 30 to 40 parts by weight of potassium, 20 to 30 parts by weight of calcium, 3 to 10 parts by weight of magnesium, and 8 to 16 parts by weight of sulfur. When the above conditions are satisfied, the amounts of the active madecassoside and asiaticoside ingredients are high.

Another aspect of the present invention pertains to a method for hydroponic cultivation of *Centella asiatica,* the method including supplying the culture medium composition for hydroponic cultivation of *Centella asiatica* described above to the roots of *Centella asiatica.*

In the method for hydroponic cultivation of *Centella asiatica* according to another aspect of the present invention, the hydroponic cultivation is performed using a deep flow technique (DFT).

In the method for hydroponic cultivation of *Centella asiatica* according to another aspect of the present invention, *Centella asiatica* is cultivated under light of 5000 to 8000k radiated in an amount of 100 to 200 µmol/m²s. When the above conditions are satisfied, the amounts of the active madecassoside and asiaticoside ingredients are high.

In the method for hydroponic cultivation of *Centella asiatica* according to another aspect of the present invention, *Centella asiatica* is cultivated under daylength conditions of 10 to 16 hours. When the above conditions are satisfied, the amounts of the active madecassoside and asiaticoside ingredients are high.

In the method for hydroponic cultivation of *Centella asiatica* according to another aspect of the present invention, carbon dioxide is supplied at a concentration of 700 to 900 ppm only during an illumination period. When the above conditions are satisfied, the amounts of the active madecassoside and asiaticoside ingredients are high.

In the method for hydroponic cultivation of *Centella asiatica* according to another aspect of the present invention, the culture medium composition for hydroponic cultivation of *Centella asiatica* is circulated from a culture medium tank to a culture medium recovery tank, and during circulation, the nutrient solution is supplied to the roots of *Centella asiatica.*

In the method for hydroponic cultivation of *Centella asiatica* according to another aspect of the present invention, the concentration of the culture medium composition is EC 0.3 to 2.8. When the above condition is satisfied, the amounts of the active madecassoside and asiaticoside ingredients are high.

In the method for hydroponic cultivation of *Centella asiatica* according to another aspect of the present invention, the concentration of the culture medium composition is increased by EC 0.3 to 0.7 at weekly intervals from a concentration of EC 0.3 to 0.7 after transplanting *Centella asiatica.* When the above conditions are satisfied, the amounts of the active madecassoside and asiaticoside ingredients are high.

Still another aspect of the present invention pertains to *Centella asiatica* produced using the method for hydroponic cultivation according to another aspect of the present invention.

Yet another aspect of the present invention pertains to an extract of *Centella asiatica* produced using the method for hydroponic cultivation according to another aspect of the present invention.

As used herein, the term "extract" is a broad concept including all materials obtained by extracting the ingredients of natural products, regardless of the extraction method, the extraction solvent, the extracted ingredient, or the form of the extract. In one embodiment, the extract may be an extract using water, a lower alcohol having 1 to 5 carbon atoms, or an aqueous solution of a lower alcohol having 1 to 5 carbon atoms.

Still yet another aspect of the present invention pertains to madecassoside isolated from *Centella asiatica* produced using the method for hydroponic cultivation according to another aspect of the present invention.

A further aspect of the present invention pertains to asiaticoside isolated from *Centella asiatica* produced using the method for hydroponic cultivation according to another aspect of the present invention.

Still a further aspect of the present invention pertains to a method of obtaining madecassoside or asiaticoside from *Centella asiatica,* the method including producing *Centella asiatica* using the method for hydroponic cultivation according to another aspect of the present invention and extracting and isolating madecassoside or asiaticoside from the produced *Centella asiatica.*

A better understanding of the present invention may be obtained through the following examples. However, these examples are merely set forth to illustrate the present invention, and are not to be construed as limiting the scope of the present invention.

### Example 1. Confirmation of culture medium composition to increase production amount

This example was conducted using a DFT hydroponic cultivation system in an artificially lit smart farm having a controllable environment. A DFT (deep-flow technique) is the most useful method in smart greenhouses (plant factories), among hydroponic cultivation methods, and is also called a water culture hydroponic method. In DFT, the roots are immersed in a culture medium (nutrient solution), and the stems are fixed with a chemically inert object to support and cultivate plants.

The light conditions for this example were set to 140±30 µmol/m²s using a fluorescent lamp (6500K, triple-wavelength fluorescent lamp). The daylength was set to 10-16 hours, and carbon dioxide was supplied at a concentration of 800 ppm only during the illumination period.

*Centella asiatica* was purchased from a *Centella asiatica* farm in Hapcheon, Gyeongnam, Korea.

In order to investigate the effect of the culture medium composition on increasing the growth of *Centella asiatica,* among runners generated from mother plants of domestic *Centella asiatica,* plants having an average weight of about 0.7 g/plant and for which the number of leaves, the size and the root development were similar were selected to thus minimize differences therebetween. Here, "runner" is the name for a stem unique to *Centella asiatica* that grows like a stem from a mother plant, creeps on the ground, and takes root at an appropriate position to thereby proliferate. Runners are also called creeping stems. Particularly, the roots and stems are grown from the nodes (growth points) of the creeping stem, which is referred to as a runner, to make daughter plants.

The selected *Centella asiatica* was cultivated through DFT after each plant was wrapped with a urethane sponge having a size of 2.5 cm × 2.5 cm × 3.0 cm so as to prevent damage to the roots and then transplanted onto a plate for hydroponic cultivation.

The composition of the culture medium used for hydroponic cultivation was composed of six macroelements mixed at the weight ratio shown in Table 1 below, along with microelements including 5.00 ppm of Fe, 0.75 ppm of B, 0.75 ppm of Mn, 0.20 ppm of Zn, 0.20 ppm of Cu, and 0.02 ppm of Mo, and the concentration of the culture medium was increased by 0.5 every week from EC (Electrical Conduction) 0.5 to EC 2.5.

**[Table 1]**

| **Amount of main ingredient (%)** | **NS 1** | **NS 2** | **NS 3** |
|---|---|---|---|
| **N** | 19-31 | 19-31 | 19-31 |
| **P** | 5-12 | 3-10 | 4-11 |
| **K** | 35-45 | 30-40 | 40-50 |
| **Ca** | 15-25 | 20-30 | 10-20 |
| **Mg** | 4-11 | 3-10 | 5-12 |
| **S** | 7-15 | 8-16 | 6-14 |
| **Total** | 100 | 100 | 100 |

*Centella asiatica* was cultivated for 6 weeks after transplanting, and harvested at the same time every week from 1 week after transplanting, and the fresh weight and the number of leaves, which are growth indicators, were observed.

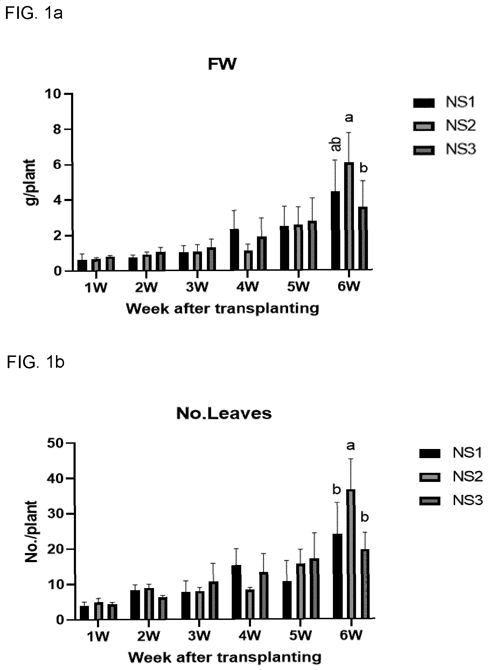

The experimental results are shown in FIGS. 1A and 1B. FIG. 1A shows the fresh weight, and FIG. 1B shows the number of leaves.

Based on the experimental results, NS2 increased 915% in fresh weight compared to 1W, showing the highest growth rate. In addition, NS1 increased 701% compared to 1W, and NS3 increased 442% compared to 1W.

The number of leaves also increased the most for NS2, which was 733% compared to 1W, followed by 600% for NS1 and 454% for NS3.

Therefore, it was confirmed that the culture medium composition of NS2 most effectively increased the fresh weight and the number of leaves.

### Example 2. Confirmation of culture medium composition to increase amounts of functional ingredients

The domestic *Centella asiatica* selected in Example 1 was cultivated for 6 weeks in the three culture media shown in Table 1 for the purpose of increasing the amounts of functional ingredients. Functional ingredients were analyzed before transplanting and after cultivation for 6 weeks for functional ingredient analysis, differently from growth indicator analysis.

Each sample was harvested for analysis, freeze-dried, and extracted, and then the amounts of madecassoside (MS) and asiaticoside (AS), which are indicator ingredients contained in *Centella asiatica,* and the total amount thereof were analyzed and compared using HPLC.

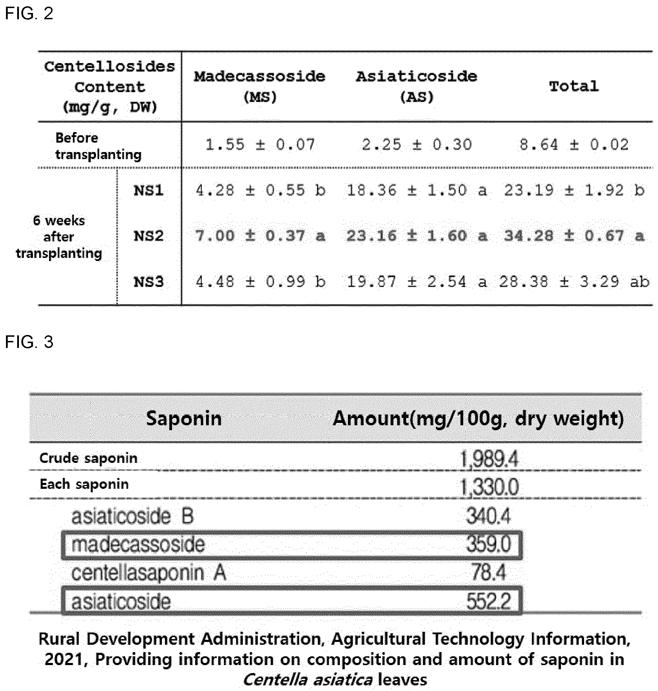

The experimental results are shown in FIG. 2.

Based on the experimental results, as shown in FIG. 2, the amounts of madecassoside and asiaticoside, and the total amount thereof were increased in all of the culture medium compositions compared to before transplanting.

For NS1, the amount of MS increased by 176%, the amount of AS increased by 716%, and the total amount thereof increased by 168%. For NS2, the amount of MS increased by 352%, the amount of AS increased by 929%, and the total amount thereof increased by 297%. For NS3, the amount of MS increased by 189%, the amount of AS increased by 783%, and the total amount thereof increased by 228%.

Comparing the culture medium compositions, for NS2, the amounts of the ingredients increased significantly compared to the other compositions. When compared with NS1 and NS3, for NS2, the amount of MS increased by 56-64%, the amount of AS increased by 17-26%, and the total amount increased by 121-48%.

In addition, when the amounts (dry weights) of the ingredients were calculated and compared with previous analysis results (Rural Development Administration, Agricultural Technology Information, 2021) for domestic *Centella asiatica* shown in FIG. 3, the amounts of MS and AS before transplanting were lower than the previous analysis results, but the amounts of MS and AS in *Centella asiatica* cultivated for 6 weeks in all three culture medium compositions were higher than the previous analysis results.

Therefore, it was confirmed that the culture medium composition developed in the present invention increased the amounts of AS and MS, which are indicator ingredients of *Centella asiatica.*

As is apparent from the above description, according to aspects of the present invention, the culture medium composition and the method for hydroponic cultivation of *Centella asiatica* using the same have an effect of speeding the growth of *Centella asiatica.*

According to aspects of the present invention, the culture medium composition and the method for hydroponic cultivation of *Centella asiatica* using the same have an effect of increasing the amounts of functional ingredients contained in *Centella asiatica.*

According to aspects of the present invention, the culture medium composition and the method for hydroponic cultivation of *Centella asiatica* using the same are capable of providing *Centella asiatica* containing madecassoside and asiaticoside at consistently high levels without being affected by the external environment. Although specific embodiments of the present disclosure have been described with reference to the accompanying drawings, those skilled in the art will appreciate that the present disclosure may be embodied in other specific forms which are in accordance with the claims. Thus, the embodiments described above should be understood to be non-limiting and illustrative in every way.

## Claims

1. A culture medium composition for hydroponic cultivation of *Centella asiatica,* **characterised in that** comprises a macroelement group comprising phosphorus (P), nitrogen (N), potassium (K), calcium (Ca), magnesium (Mg), and sulfur (S), a weight ratio of phosphorus (P), nitrogen (N), potassium (K), calcium (Ca), magnesium (Mg), and sulfur (S) being 1:3.0-6.0:4.8-7.0:3.0-5.2:0.4-1.7:1.0-3.0.

2. The culture medium composition of claim 1, wherein the composition comprises a microelement group comprising iron, boron, manganese, zinc, copper, and molybdenum,
the microelement group comprises 3.00 to 7.00 ppm of iron, 0.30 to 1.00 ppm of boron, 0.30 to 1.00 ppm of manganese, 0.10 to 0.30 ppm of zinc, and 0.01 to 0.05 ppm of molybdenum, based on a total weight of the composition.

3. The culture medium composition of claim 1, wherein the macroelement group comprises, based on 100 parts by weight thereof, 19 to 31 parts by weight of nitrogen, 3 to 10 parts by weight of phosphorus, 30 to 40 parts by weight of potassium, 20 to 30 parts by weight of calcium, 3 to 10 parts by weight of magnesium, and 8 to 16 parts by weight of sulfur.

4. A method for hydroponic cultivation of *Centella asiatica,* comprising supplying the culture medium composition of any one of claims 1 to 3 to roots of *Centella asiatica.*

5. The method of claim 4, wherein the hydroponic cultivation is performed using a deep flow technique (DFT),
the method is performed under light of 5000 to 8000k radiated in an amount of 100 to 300 µmol/m²s.

6. The method of claim 4 or 5, wherein the method is performed under daylength conditions of 10 to 16 hours,
carbon dioxide is supplied at a concentration of 700 to 900 ppm only during an illumination period.

7. The method of one of claims 4 to 6, wherein the culture medium composition is circulated from a culture medium tank to a culture medium recovery tank, and a nutrient solution is supplied to the roots of *Centella asiatica* during circulation.

8. The method of one of claims 4 to 7, wherein a concentration of the culture medium composition is EC 0.3 to 2.8, wherein the concentration of the culture medium composition is increased by EC 0.3 to 0.7 at weekly intervals from a concentration of EC 0.3 to 0.7 after transplanting *Centella asiatica.*

9. A method for obtaining *madecassoside* or *asiaticoside* from *Centella* asiatica, comprising supplying the culture medium composition of anyone of claims 1 to 3 to roots of Centella asiatica and extracting and isolating madecassoside or asiaticoside from the produced Centella asiatica

## Patentansprüche

1. Kulturmediumzusammensetzung zur hydroponischen Kultivierung von *Centella asiatica,* **dadurch gekennzeichnet, dass** die Kulturmediumzusammensetzung eine Makroelementgruppe umfasst, die Phosphor (P), Stickstoff (N), Kalium K), Calcium (Ca), Magnesium (Mg) und Schwefel (S) umfasst, wobei ein Gewichtsverhältnis von Phosphor (P), Stickstoff (N), Kalium (K), Calcium (Ca), Magnesium (Mg) und Schwefel (S) 1 : 3,0 bis 6,0 : 4,8 bis 7,0 : 3,0 bis 5,2 : 0,4 bis 1,7 : 1,0 bis 3,0 beträgt.

2. Kulturmediumzusammensetzung nach Anspruch 1, wobei die Zusammensetzung eine Mikroelementgruppe umfasst, die Eisen, Bor, Mangan, Zink, Kupfer und Molybdän umfasst,
wobei die Mikroelementgruppe bezogen auf ein Gesamtgewicht der Zusammensetzung 3,00 bis 7,00 ppm Eisen, 0,30 bis 1,00 ppm Bor, 0,30 bis 1,00 ppm Mangan, 0,10 bis 0,30 ppm Zink und 0,01 bis 0,05 ppm Molybdän umfasst.

3. Kulturmediumzusammensetzung nach Anspruch 1, wobei die Makroelementgruppe bezogen auf 100 Gewichtsteile derselben 19 bis 31 Gewichtsteile Stickstoff, 3 bis 10 Gewichtsteile Phosphor, 30 bis 40 Gewichtsteile Kalium, 20 bis 30 Gewichtsteile Calcium, 3 bis 10 Gewichtsteile Magnesium und 8 bis 16 Gewichtsteile Schwefel umfasst.

4. Verfahren zur hydroponischen Kultivierung von *Centella asiatica,* umfassend:
Versorgen von Wurzeln von *Centella asiatica* mit der Kulturmediumzusammensetzung nach einem der Ansprüche 1 bis 3.

5. Verfahren nach Anspruch 4, wobei die hydroponische Kultivierung mittels einer Tiefwasser-Nährstoff-Film-Technik (DFT, eng. *deep flow technique*) durchgeführt wird,
wobei das Verfahren bei einer Lichtstärke von 5000 bis 8000 k und einer Strahlungsintensität von 100 bis 300 µmol/m²s durchgeführt wird.

6. Verfahren nach Anspruch 4 oder 5, wobei das Verfahren unter Tageslängebedingungen von 10 bis 16 Stunden durchgeführt wird,
wobei nur während eines Beleuchtungszeitraums Kohlenstoffdioxid in einer Konzentration von 700 bis 900 ppm zugeführt wird.

7. Verfahren nach einem der Ansprüche 4 bis 6, wobei die Kulturmediumzusammensetzung von einem Kulturmediumbehälter zu einem Kulturmediumsammelbehälter zirkuliert wird und die Wurzeln von *Centella asiatica* während der Zirkulation mit einer Nährstofflösung versorgt werden.

8. Verfahren nach einem der Ansprüche 4 bis 7, wobei eine Konzentration der Kulturmediumzusammensetzung EC 0,3 bis 2,8 beträgt, wobei die Konzentration der Kulturmediumzusammensetzung nach dem Umsetzen von *Centella asiatica* ausgehend von einer Konzentration von EC 0,3 bis 0,7 in wöchentlichen Abständen um EC 0,3 bis 0,7 gesteigert wird.

9. Verfahren zur Gewinnung von Madecassosid oder Asiaticosid aus *Centella asiatica,* umfassend: Versorgen von Wurzeln von *Centella asiatica* mit der Kulturmediumzusammensetzung nach einem der Ansprüche 1 bis 3 und Extrahieren und Isolieren von Madecassosid oder Asiaticosid aus der produzierten *Centella asiatica.*

## Revendications

1. Composition de milieu de culture pour la culture hydroponique de *Centella asiatica,* **caractérisée en ce que** la composition de milieu de culture comprend un groupe de macroéléments comprenant le phosphore (P), l'azote (N), le potassium (K), le calcium (Ca), le magnésium (Mg) et le soufre (S), un rapport de poids du phosphore (P), de l'azote (N), du potassium (K), du calcium (Ca), du magnésium (Mg) et du soufre (S) étant de 1 : 3,0 à 6,0 : 4,8 à 7,0 : 3,0 à 5,2 : 0,4 à 1,7 : 1,0 à 3,0.

2. Composition de milieu de culture selon la revendication 1, dans laquelle la composition comprend un groupe de microéléments comprenant le fer, le bore, le manganèse, le zinc, le cuivre et le molybdène,
le groupe de microéléments comprend 3,00 à 7,00 ppm de fer, 0,30 à 1,00 ppm de bore, 0,30 à 1,00 ppm de manganèse, 0,10 à 0,30 ppm de zinc et 0,01 à 0,05 ppm de molybdène par rapport à un poids total de la composition.

3. Composition de milieu de culture selon la revendication 1, dans laquelle le groupe de macroéléments comprend, par rapport à 100 parties en poids de celui-ci, 19 à 31 parties en poids de l'azote, 3 à 10 parties en poids de phosphore, 30 à 40 parties en poids de potassium, 20 à 30 parties en poids de calcium, 3 à 10 parties en poids de magnésium et 8 à 16 parties en poids de soufre.

4. Procédé pour la culture hydroponique de *Centella asiatica,* comprenant l'étape consistant à fournir la composition de milieu de culture selon l'une quelconque des revendications 1 à 3 à des racines de *Centella asiatica.*

5. Procédé selon la revendication 4, dans laquelle la culture hydroponique est réalisée en utilisant un système DFT (anglais : *deep flow technique*),
le procédé est réalisé sous une lumière de 5000 à 8000 k irradiée dans une quantité de 100 à 300 µmol/m²s.

6. Procédé selon la revendication 4 ou 5, dans laquelle le procédé est réalisé à des conditions de durée de jour de 10 à 16 heures,
du dioxyde de carbone est fourni à une concentration de 700 à 900 ppm seulement pendant une période d'éclairage.

7. Procédé selon l'une quelconque des revendications 4 à 6, dans laquelle la composition de milieu de culture est circulée d'un réservoir de milieu de culture à un réservoir collecteur de milieu de culture et une solution nutritive est fournie aux racines de *Centella asiatica* pendant la circulation.

8. Procédé selon l'une quelconque des revendications 4 à 7, dans laquelle une concentration de la composition de milieu de culture est d'EC 0,3 à 2,8, dans laquelle la concentration de la composition de milieu de culture est augmentée par EC 0,3 à 0,7 à des intervalles hebdomadaires à partir d'une concentration d'EC 0,3 à 0,7 après la transplantation de *Centella asiatica.*

9. Procédé pour obtenir du madécassoside ou de l'asiaticoside à partir de *Centella asiatica,* comprenant les étapes consistant à fournir la composition de milieu de culture selon l'une quelconque des revendications 1 à 3 à des racines de *Centella asiatica* et extraire et isoler du madécassoside ou de l'asiaticoside de la *Centella asiatica* produite.
